# EUROPEAN PATENT APPLICATION

(11) **EP 4 316 576 A1**
(43) Date of publication of application: **07.02.2024**
(21) Application number: 22188886.0
(22) Date of filing: 04.08.2022
(51) Int. Cl.: A61N 5/06

(54) **ADAPTIVE PHOTOBIOMODULATION (PBM)**

(71) Applicant: Seaborough Life Science B.V., 1098XG Amsterdam (NL)
(72) Inventor: BERENDS, Anne Claire, 3551 TJ UTRECHT (NL); KRAMES, Michael, KENTFIELD, 94904 (US)
(74) Representative: Hoyng Rokh Monegier B.V.

(57) **Abstract**

An electro-optical device (1) having a radiation unit (10) for emitting a pulsed radiation beam (11) having a peak emission wavelength between 610-1400nm, a detection unit (20) for detecting a predetermined area of the body of a user, and a radiation control unit (30) for receiving an input from the detection unit. The electro-optical device (1) is adapted to direct the radiation beam (11) towards the predetermined area using the radiation control unit (30), such that the radiation beam has a peak irradiation intensity above 0.1 mW/cm² at a surface of the body of the user in the predetermined area.

## Description

### TECHNICAL FIELD

The invention relates to a device that delivers radiation sufficient to induce a photobiomodulation (PBM) effect.

### BACKGROUND ART

Photobiomodulation (PBM) involves irradiating a living organism at certain energy/power levels to induce biological or biochemical responses. The irradiation may be in the visible spectrum, such as red light, or in the non-visible spectrum, such as infrared (IR). There has been a significant amount of research about the medical benefits of employing PBM therapy to treat physical and psychological symptoms.

However, most of the equipment that administer PBM radiation near-field excitation of the skin or treatment area (i.e. proximity devices) or use very high powers to irradiate the entire body. These specifications render the PBM effect exclusively in specialized medical or therapeutic devices that are often bulky and/or expensive. These factors greatly limit the availability of the medical benefits of PBM within the general public.

In Applicant's PCT applications published as WO 2020/119965 and WO 2021/099642, herewith incorporated by reference in their entirety, a concept of incorporating PBM into general lighting has been proposed, using a lighting apparatus for emitting stable visible light and additionally emitting pulsed PBM radiation. By driving a radiation source with a pulse instead of a continuous wave (or nearly continuous wave) signal, it is possible to boost the peak power during the pulse to achieve a PBM response. This allows the general lighting apparatus to emit a sufficient amount of power to enable a certain power density (irradiance) at a certain distance that may induce PBM responses. A sensor is provided in such a lighting apparatus to turn on or off PBM radiation, depending on the presence and/or distance of the user.

However, in order to emit an amount of radiation sufficient to induce a PBM response, the known PBM devices still require a considerable amount of energy to induce a PBM response and require a significant amount of time to achieve a desired dose level.

### SUMMARY OF THE INVENTION

It is an objective of the present invention to provide an apparatus that is more efficient with regard to the energy needed to induce a PBM response and with regard to the time needed to achieve the desired dose level.

The first aspect of the present invention relates to an electro-optical device. The device may comprise a radiation unit adapted to emit a radiation beam, a detection unit adapted to detect a predetermined area of the body of a user (e.g. the user's face), and a radiation control unit for receiving an input from the detection unit. The electro-optical device may be adapted to direct the radiation beam towards the predetermined area using the radiation control unit, such that the radiation beam has a peak irradiation intensity above 0.1 mW/cm² at a surface of the body of the user in the predetermined area.

The invention is based on an insight that well known detection methods (such as facial detection) may be utilized to give useful information regarding not only the presence of the user as such, but also the relative positions between the device and the user, e.g. the direction and/or distance from the device to the user. Using such information, the electro-optical device can direct the radiation beam towards the predetermined area in a more directed and/or focused manner, while unnecessary radiation outside the predetermined area is reduced and less energy is wasted. This achieves the goal described above.

The radiation control unit may be adapted to control the radiation unit to adaptively adjust a radiation pattern of the radiation beam (e.g. beam direction, beam angle, focus, or combination of these) in dependence on the input from the detection unit. In this way, the electro-optical device can adaptively adjust the radiation pattern beyond simply switching on and off the entire PBM radiation, to adequately generate a PBM response in an efficient manner. Optionally, the radiation beam may be pulsed, as described in WO 2020/119965 and WO 2021/099642.

In a preferred embodiment, the predetermined area covers at least a portion of the face of the user. This has several advantages. First, facial detection method is well-known and well-developed, which makes implementation simpler and less expensive. Secondly, the skin on the face is usually not covered by clothes and provides relatively large area (average surface area of a male face is 1323 cm²). By directing the radiation beam towards at least a portion of the face of the user, PBM effect could be achieved in a particularly efficient way.

The radiation pattern refers to the manner (e.g. beam angle, direction, illuminated area(s), transmission power, etc) in which the radiation beam is being emitted. Controlling the radiation pattern enables the electro-optical device to steer the radiation beam towards the predetermined area of the user even when the user moves. In this way, less energy is wasted to induce a PBM response, and the condensed energy in the predetermined area also enables the desired dose level to be achieved faster. In an embodiment, the radiation control unit is adapted to adjust the radiation pattern to cover substantially only the predetermined area.

The radiation pattern may be controlled in various ways. For example, the radiation pattern may be conditioned (e.g., steered, focused, etc.) by an optical element (e.g. lens(es), mirror(s), etc). Alternatively or additionally, a multiple-element emitter may be used (e.g. using plural LEDs or VCSEL array in the radiation unit). Each radiation element in the multiple-element emitter may be arranged to map to an effective illumination region. When the predetermined area is within the effective illumination region, that radiation element is switched on. When the predetermined area is not within the effective illumination region, that radiation element is not switched on (e.g. switched off by default). This way, the electro-optical device can map its light out to the desired location without requiring any moving parts.

In an embodiment, the radiation unit is adapted to project the radiation beam in a first direction. The radiation control unit may be adapted to adjust the first direction and/or spread angle of the radiation beam in reaction to an input from the detection unit, so as to adjust the radiation pattern of the radiation beam. This enables a simple implementation, allowing the direction of the radiation beam to simply be changed when the user is detected or moves.

The first direction of the radiation beam may refer to the direction of the nominal centerline of the beam. The spread angle of the radiation beam may refer to the angle between the two directions for which the intensity is 50% of the maximum intensity as measured in a plane through the nominal beam centerline, sometimes referred to as full-angle-at-half power (FAHP). For beams that do not possess rotational symmetry, the beam angle is typically given for two planes at 90 degrees, called the maximum and minimum angles. In the context of the present application, the spread angle refers to the minimum angle in this situation. Note that according to the present invention, the radiation pattern may comprise emitting the radiation beam in plural directions towards plural predetermined areas (e.g. in the case of plural users). In this case the spread angle may be independent in each direction.

In an embodiment, the radiation unit comprises an optical element comprising one or more lenses, and/or one or more mirrors, and/or one or more diffraction optical elements, and wherein the radiation control unit is adapted to move and/or rotate a part of the optical element to adjust the first direction of the radiation beam.

The radiation unit may be driven by a driving current, and the radiation control unit may be adapted to adjust a pulse width, and/or a frequency, and/or an amplitude, of the driving current, in reaction to an input from the detection unit, so as to adjust the radiation pattern of the radiation beam. In a multiple-element emitter discussed above, this adjustment may be adjusted at the level of individual radiation elements.

In an embodiment, the radiation unit comprises a plurality of radiation elements, each driven by a driving current, and wherein the radiation control unit is adapted to adjust the driving current for individual radiation elements in reaction to an input from the detection unit to adjust the radiation pattern of the radiation beam. In this way, the radiation pattern can be adjusted without relying on moving or rotating one or more optical elements.

In an embodiment, the radiation unit comprises a plurality of LEDs, and the radiation control unit is adapted to switch on or switch off a subset of the plurality of LEDs in reaction to an input from the detection unit, so as to adjust the radiation pattern of the radiation beam. This enables switching on and switching off a subset of the plurality of LEDs based on the detection of the predetermined area. Each subset of LEDs may comprise one or more LEDs, and different subsets may comprise different numbers of LEDs.

Additionally or alternatively, the radiation unit may comprise a vertical cavity surface emitting laser (VCSEL) array having one or more radiation elements, each radiation element having an effective illumination region, wherein, for each radiation element, the radiation control unit is adapted to switch on the radiation element when the predetermined area is within the effective illumination region, and does not switch on the radiation element when the predetermined area is not within the effective illumination region. Compared to LEDs, VCSEL arrays have an advantage of providing narrower beam angles, allowing radiation energy to be concentrated on a useful area at larger distances. Additional flexibility may also be achieved by moving and/or rotating one or more deflection members (such as mirrors or (micro)lenses) integrated in the VCSEL array, which may function as (part or the entirety of) the optical element described above.

In an embodiment, the detection unit is adapted to determine a distance between the electro-optical device and the predetermined area and generate an output in dependence on said distance, and the radiation control unit is adapted to react to the detection unit in dependence on the output of the detection unit. The distance may be determined based on a dedicated distance sensor, or may be estimated based on the size of the predetermined area detected.

Using one or more measures discussed above, alone or in combination, a varying degree of flexibility of radiation pattern adjustment can be achieved. This enables a wide range of PBM applications that are accessible to the user in their daily life.

The radiation unit may be adapted to emit a pulsed radiation beam having a peak emission wavelength between 610-1400 nm. In an embodiment, the peak emission wavelength is in the (near) infrared ((N)IR) region, such as 700-1400 nm. In an embodiment this range may be 760-1400 nm or 800-1100 nm. Another option is the range 800-870 nm. The peak emission wavelength may also be with the spectrum of red light such as the range 610-700 nm. Irradiating the predetermined area (e.g. user's face) with radiation in these spectrums can induce beneficial PBM responses. Some embodiments may utilize devices emitting in different wavelength regimes simultaneously.

It is generally accepted that an irradiation intensity of > 0.1 mW/cm² at the skin's surface is required to induce a meaningful PBM effect. According to embodiments of the present invention, the electro-optical device is designed to direct the radiation beam towards the predetermined area using the radiation control unit, such that the radiation beam has a peak irradiation intensity above 0.1 mW/cm², preferably above 1 mW/cm² at a surface of the body of the user in the predetermined area. This makes it possible to efficiently inducing PBM effect in the predetermined area.

In an embodiment, the electro-optical device is adapted to be placed on a desk or a table. For example, the electro-optical device may be embodied in a desk accessory that emits the radiation beam. The desk or table is an ideal place to put a PBM device, as it has well-defined dimensions and thereby a predictable distance between the PBM device and the user. This enables the radiation unit (e.g. transmission power of the radiation beam, etc.) of the electro-optical device to be designed in view of the typical desk/table dimensions. In this way, a reliable, efficient PBM response could be achieved in a simple and reliable manner.

Such a desk accessory may be with or without any other electrical functionality (e.g. general lighting functionality or display functionality). The electro-optical device may be arranged as a 'PBM gadget' that is placed on the desk solely or primarily for the PBM purpose (the device may include additional non-electrical functionalities such as serving as a pencil holder). For example, without the general lighting functionality, hence without the stem and head of the lamp in the user's face, these objects can be placed much closer to the user. This relatively short distance enables a relatively large radiation intensity to be obtained in the predetermined area, allowing the desired dose level to be achieved faster.

In an embodiment, the electro-optical device is mounted on a ceiling or a wall, and/or incorporated in a portable user equipment (such as a smartphone) and/or a display apparatus (such as a television). Also these embodiments allow the electro-optical device to utilize the typical distances between such devices and the user, in combination with one or more radiation pattern adjustment measures, to reliably induce the PBM effect.

In an embodiment, the electro-optical device is incorporated in a general lighting apparatus, wherein the radiation unit is adapted to project the radiation beam in a spread angle within 2×30°, preferably within 2×25°, more preferably within 2×20°, more preferably within 2×15°, more preferably within 2×10°. As discussed above in the background section, the idea of incorporating a PBM device in a general lighting apparatus as such is known. But taking advantage of the radiation pattern adjustment capability of the present invention and the relatively large typical distance between lighting devices and the user, a particular narrow spread angle for the radiation beam (e.g. 2×10°) can be adopted. Using such a narrow beam, the efficiency in both energy use and dose accumulation can be obtained.

More generally, in the electro-optical device according to the invention (not limited to general lighting devices), the radiation unit may be adapted to project the radiation beam in a spread angle (or FAHP) within +/- 30°, preferably within +/- 25°, more preferably within +/- 20°, more preferably within +/- 15°, more preferably within +/- 10° or less about the center beam line.

The electro-optical device may further comprise a timer which starts in reaction to an input from the detection unit, and the radiation control unit is adapted to stop emitting the radiation beam from the radiation unit once the timer expires. The radiation control unit may be adapted to set the timer in accordance with a predetermined expiration time, or to set the timer such that the timer expires after a predetermined dose level (e.g. in the range 0.01-50 J/cm², preferably in the range 0.1-20 J/cm², more preferably in the range 1-10 J/cm², more preferably in the range 4-7 J/cm²) in the predetermined area is exceeded. This enables efficient dose control. Moreover, in combination with the measures discussed below, personalized dose control for individual users can be achieved.

The detection unit may be adapted to detect multiple predetermined areas corresponding to multiple users. This allows the electro-optical device to direct the radiation beam towards multiple users in turn or at the same time.

In a preferred embodiment, the detection unit is adapted to recognize and distinguish between individual users (e.g. using facial recognition or similar methods to determine the user identity), and for each recognized user, the radiation control unit is adapted to direct the radiation beam towards a predetermined area in the body of said user by adjusting the radiation pattern, such that in each predetermined area for each user, the radiation beam has a peak irradiation intensity above 0.1 mW/cm² preferably above 1 mW/cm² (at the same time or at different times). This enables the electro-optical device to induce PBM response on multiple users and makes it possible to control the dose for each user individually. By identifying specific users, the electro-optical device can easily keep track of each user even if the users move or swap positions. This also makes it possible to reliably regulate the dose for each specific user.

In an embodiment, for each recognized user, the electro-optical device is adapted to start a timer upon recognizing the user by the detection unit, and the radiation control unit is adapted to adjust the radiation pattern upon expiration of each individual timer on a user-by-user basis. For example, when the timer for a particular user expires (indicating that the user in question has obtained an accumulated dose level, e.g. in a range 0.01-50 J/cm² , preferably in the range 0.1-20 J/cm², more preferably in the range 1-10 J/cm², more preferably in the range 4-7 J/cm²), the radiation control unit may stop directing the radiation beam towards that particular user.

In an embodiment, for each recognized user, the radiation control unit is adapted to pause the corresponding timer when the user is no longer recognized (e.g. when the user leaves the room, is temporarily blocked, or moves too far away), and resume the timer when the user is recognized again. As the electro-optical device recognizes each specific user, this enables the electro-optical to keep of the accumulated dose level for every user, even if some user temporarily disappears and reappears.

### BRIEF DESCRIPTION OF THE DRAWINGS

Embodiments will now be described, by way of example only, with reference to the accompanying schematic drawings in which corresponding reference symbols indicate corresponding parts, and in which:
Figs. 1A and 1B schematically show embodiments of the electro-optical device 1 according to the present invention.
Figs. 2A-2D schematically show various devices in which the electro-optical device 1 according to the present invention may be implemented.
Figs. 3A-3D schematically show embodiments in which the radiation pattern is controlled using optical means.
Figs. 4A-4C schematically show embodiments in which the radiation pattern is controlled by controlling a radiation source.
Fig. 5 schematically shows an embodiment of an electro-optical device 1 according to the present invention, which comprises a timer for the purpose of dose control.
Fig. 6 schematically shows an embodiment in which the electro-optical device 1 is capable of recognizing and distinguishing between individual users.

### DESCRIPTION OF ILLUSTRATIVE EMBODIMENTS

The following is a description of certain embodiments of the invention, given by way of example only and with reference to the drawings.

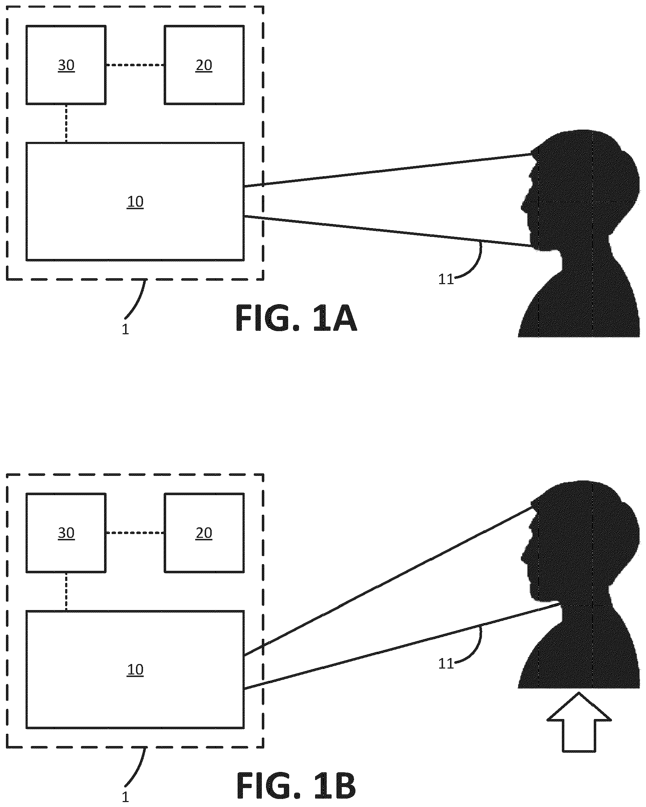

Fig. 1A shows an embodiment of an electro-optical device 1 according to the present invention. The device 1 may comprise a radiation unit 10 for emitting a radiation beam 11, a detection unit 20 for detecting an area of interest, and a radiation control unit 30 for receiving an input from the detection unit 20.

The detection unit 20 functions to detect a predetermined area of the body of a user. The predetermined area may relate to any part of the user's body. In a preferred embodiment, the predetermined area covers at least a portion of the face of the user, as shown in Fig. 1A.

Facial detection or recognition is a type of object detection, which is widely used in smart phones, security systems, photography, video conferences, lip reading, etc. One method relies on constructing a feature vector based on the detected size and location of features such as eyes, mouth and nose. For face recognition, the face-features are not only detected and recognized as a face-feature in general, but the face-features of a particular person are identified, e.g. by determining their specific size and (relative) position and comparing these with a reference image. The present invention may be implemented using facial detection or facial recognition, while the latter is advantageous when multiple users are involved or for personal dose control.

There are various well-known technologies used for facial detection or recognition. Examples are conventional image analysis using visible light images and/or (N)IR images, Human identification at a distance (HID), 3D face recognition, thermal images, etc. The present invention may be implemented using any of these technologies, a combination of these, or any other technologies that can detect or recognize a user's face.

Utilizing facial detection or recognition in embodiments of the present invention has another advantage. The skin on the face is usually not covered by clothes and provides relatively large area (average surface area of a male face is 1323 cm²). By directing the radiation beam 11 towards at least a portion of the face of the user, PBM effect can be induced in this relatively large and stable area. This improves the efficiency of the PBM device.

In this way, well known detection methods (such as facial detection) may be utilized to give useful information regarding the relative positions between the device and the user, e.g. the direction and/or distance from the device to the user. As shown in Fig. 1A, using such information, the electro-optical device can direct the radiation beam towards the predetermined area in a directed and/or focused manner, reducing unnecessary radiation outside the predetermined area. This may be achieved by placing the electro-optical device 1 at a designed place in combination with a designed beam angle (e.g. with a full-angle-at-half power (FAHP) within 2×30°, 2×25°, 2×20°, 2×15°, or 2×10°, depending on the designed location of the electro-optical device 1), and/or by adjusting the radiation pattern of the radiation beam 11 as described below.

The radiation control unit 30 may be adapted to control the radiation unit to adaptively adjust a radiation pattern of the radiation beam (e.g. beam direction, beam angle, focus, or combination of these) in dependence on the input from the detection unit. An embodiment is shown in Fig. 1B.

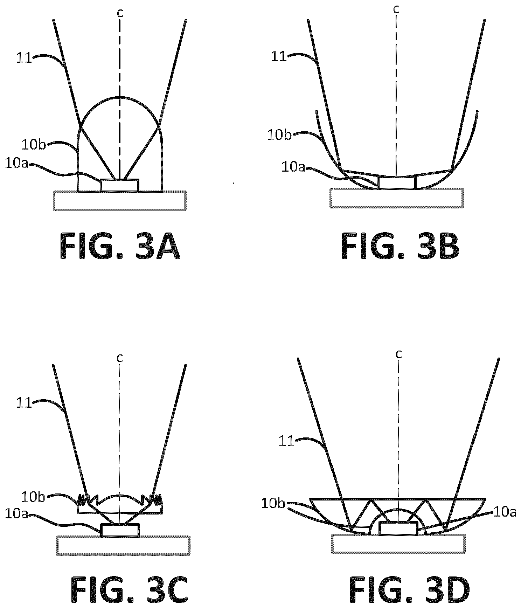

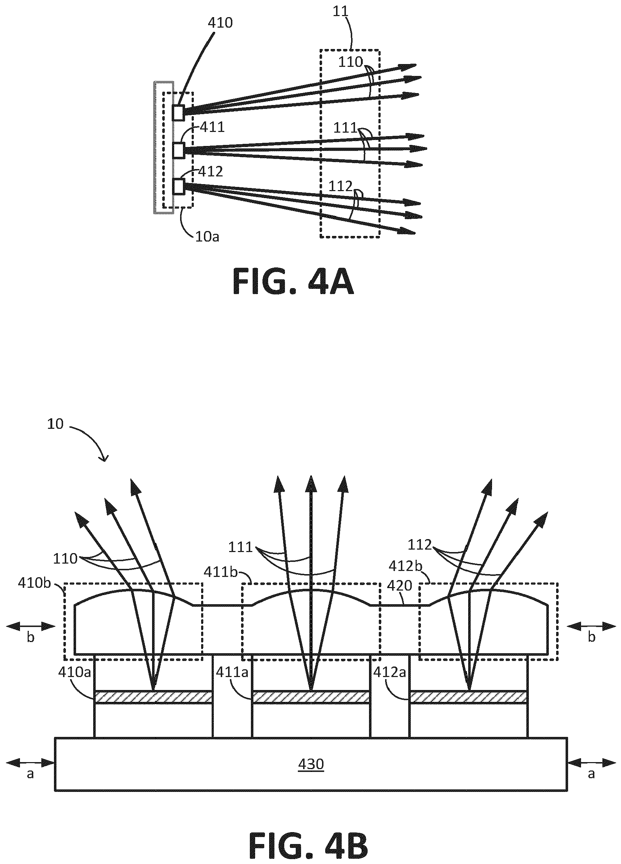

As shown in Fig. 1B, when the user moves (e.g. stands up from their chair), the radiation pattern is adjusted to direct the radiation beam 11 towards the new location of the predetermined area (e.g. face). The radiation pattern may be adjusted by adjusting the direction of the beam (as shown in the figure) or in another way (e.g. by changing the spreading angle, focus, etc, see below Figs. 3A-3D and 4A-4C).

In an embodiment, the detection unit is adapted to determine a distance between the electro-optical device and the predetermined area generate an output in dependence on said distance, and the radiation control unit is adapted to react to the detection unit in dependence on the output of the detection unit. The distance may be determined based on a dedicated distance sensor (not shown), or may be determined using the detection unit 20. For example, the detection unit 20 may estimate the size of the predetermined area and on the basis of this estimate a distance.

The radiation unit may be adapted to emit a radiation beam having a peak emission wavelength between 610-1400 nm. In an embodiment, the peak emission wavelength in in the (near) infrared ((N)IR) region, such as 700-1400 nm. In an embodiment this range may be 760-1400 nm or 800-1100 nm. Another option is the range 800-870 nm. The peak emission wavelength may also be with the spectrum of red light such as the range 610-700 nm. Recent advances in medical research have demonstrated that irradiating a living organism with radiation comprising the (N)IR spectrum and/or red light at certain energy/power levels may induce beneficial biological or biochemical responses. Such irradiation is often referred to as photobiomodulation (PBM). Available medical research results on the medical benefits of employing PBM therapy to treat physical and psychological symptoms are rapidly increasing. Some wavelengths that have attracted particular attention include 606, 627, 630, 632.8, 640, 660, and 670 nm (in the red region) and 785, 800, 804, 808, 810, 820, 830, 850, 904, 980 and 1060 nm (in the NIR region). Some spectral ranges that have attracted particular attention include 650-680 and 800-870 nm. The radiation unit may be configured to emit radiation in multiple wavelength regimes simultaneously.

The radiation beam 11 may be pulsed, as described in WO 2020/119965 and WO 2021/099642. With a pulse instead of a constant or nearly constant signal, it is possible to boost the peak radiant power emitted by the light source while consuming the same amount of electric power. In other words, a light source with pulsed emission can achieve a much higher peak radiant power than the same device with continuous wave (CW) emission. It is generally accepted that an irradiation intensity of > 0.1 mW/cm² at the skin's surface is required to induce a meaningful PBM effect. According to embodiments of the present invention, the electro-optical device is designed to direct the radiation beam towards the predetermined area using the radiation control unit, such that the radiation beam has a peak irradiation intensity above 0.1 mW/cm², preferably 1 mW/cm² or more at a surface of the body of the user in the predetermined area, which can efficiently induce PBM effect and makes it possible to obtain the desired dose level (e.g. 0.01-50 J/cm², preferably in the range 0.1-20 J/cm², more preferably in the range 1-10 J/cm², more preferably in the range 4-7 J/cm²) within a few hours.

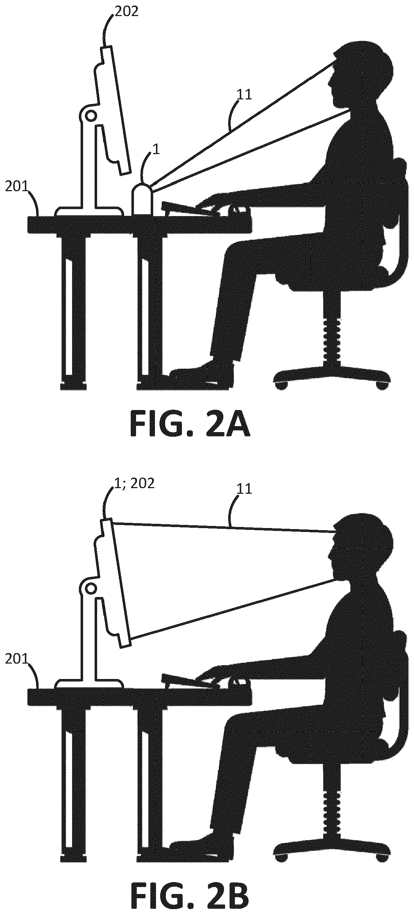

Fig. 2A-2D show various examples of how the electro-optical device 1 according to the present invention may be applied. Such devices may contain measures described above with respect to Figs. 1A and 1B.

Fig. 2A shows an embodiment in which the electro-optical device 1 is designed to be placed on a desk 201 (or a table), such as in the form of a desk accessory. A desk (or table) has well-defined dimensions. This makes it is an ideal place to put a PBM device as these dimensions set the boundary of the distance between the electro-optical device 1 and the user. Taking these dimensions into account, this makes it possible to design the characteristic of the radiation beam 11 (e.g. spreading angle, etc) so that the width of the radiation beam 11 is just enough to cover the predetermined area, with sufficient power to induce PBM effect.

Many people have objects on their desk (e.g. pencil holder, sound speakers, etc). These objects are suitable for incorporating the electro-optical device 1. Most of these objects are placed relatively close to the user, making these objects suitable for embodying the PBM functionality.

Fig. 2B shows an embodiment in which the electro-optical device 1 is embodied in a computer display 202. Similar to Fig. 2A, this embodiment enables the typical dimensions of a desk 201 (or a table) to be utilized to design the electro-optical device 1 so that the transmission power of the radiation beam is high enough to induce PBM response during intended use, taking into account the propagation of the radiation beam 11. In the embodiment shown, the radiation beam 11 focuses on the user's face (instead of spreading), which allows the energy of the radiation beam to be efficiently used to induce PBM effect. The focus (and/or direction of the beam) may be adjusted when the user moves further or closer, as part of the radiation pattern adjustment. Similarly, the electro-optical device 1 may be embodied in a television (not shown), where the user may be assumed to sit in a certain distance away. Also, the radiation unit may be incorporated into a separate devices that mounts onto or nearby the computer display or television.

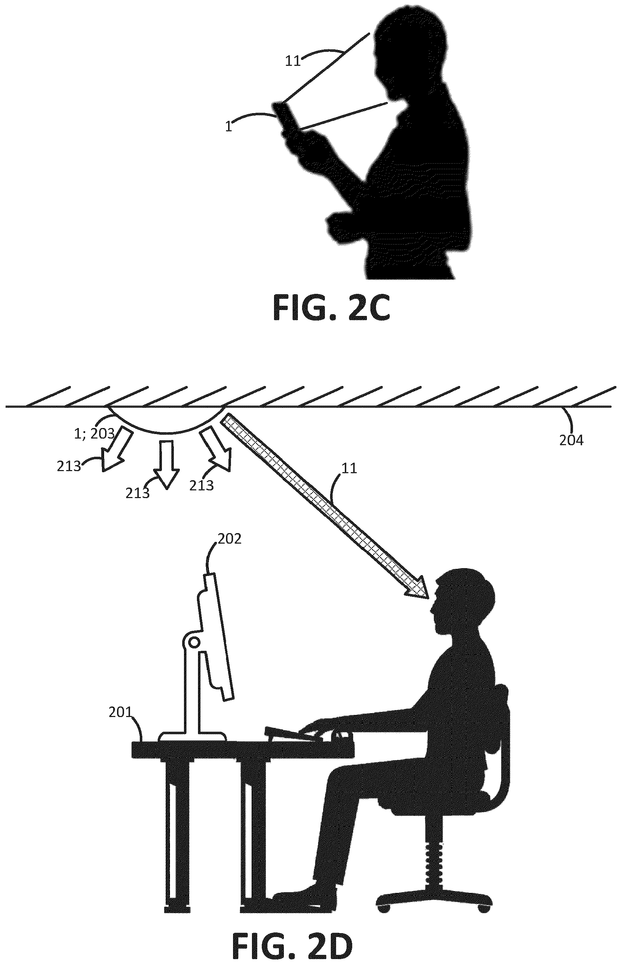

Fig. 2C shows an embodiment in which the electro-optical device 1 is incorporated in a portable user equipment, such as a smartphone. This embodiment has several advantages. First, the facial detection / recognition functionality is usually already implemented in such devices, which reduces the cost of implementing the present invention. Moreover, many users have a relatively long screentime with such devices and keep a relatively short and stable distance with the screen (e.g. 30 cm). This makes such portable devices an ideal equipment for providing PBM effect.

Fig. 2D shows an embodiment in which the electro-optical device 1 is incorporated in a general lighting apparatus 203. In the embodiment shown, the general lighting apparatus 203 is mounted on a ceiling 204. It may also be mounted a wall, or a self standing lamp, or any other lamp arrangement.

In addition to the radiation beam 11, the general lighting apparatus 203 additionally emits visible light 213 for the purpose of general lighting, as described in WO 2020/119965 and WO 2021/099642. In contrast to the prior art, the present invention allows the radiation beam 11 to be much more directed and/or focused. Preferably, the radiation beam 11 has a spread angle within 2×30°, preferably within 2×25°, more preferably within 2×20°, more preferably within 2×15°, more preferably within 2×10°. As can be seen, while the visible light 213 spreads in a wide angle for the general light purpose, the radiation beam 11 is projected onto the predetermined area (e.g. user's face) so as to induce PBM effect in that area. This allows energy saving and optimal dosing. The better the delivery of the radiation beam 11 is controlled, the less energy is 'lost' so that and the user receives exactly the right amount of PBM radiation.

The table below shows simulation results reflecting this advantage with respect to a comparative example. The embodiment and the comparative example use the same pulse condition for the radiation beam (current, frequency, pulse width, duty cycle), while using a significantly narrower beam angle in the embodiment, and hence the illuminated area. In the simulated embodiment, the illuminated area is barely larger than the average surface area of a male face (1323 cm²; reference: Jayasekara et al. Forensic Med. Anat. Res. 2016). As such, most of the radiation energy is condensed in the predetermined area, and accordingly the time needed to obtain an accumulated dose of 6.3J cm² is significantly shorter (1.2 hours vs. more than 8 hours), and considerably fewer LEDs can be used. Consequently, the amount of energy needed is significantly lower in the embodiment.

| | Embodiment | Comparative Example |
|---|---|---|
| Number of LEDs | 7 | 28 |
| Beam angle | 20° | 90° |
| Illuminated area (half intensity area) at 1.3m distance | 1650 cm² | 5.31 m² |
| Time needed for 6.3 J/cm² dose (with 100 Hz pulsing, 10% duty cycle, 3578 mW peak output power) | 1.2h | > 8h |

### Radiation pattern adjustment

Figs. 3A-3D show several embodiments in which the radiation pattern is controlled using an optical element 10b. These measures may be implemented in the electro-optical device 1 in Figs. 1-2.

In these embodiments, the radiation unit 10 comprises a radiation source 10a and may optionally comprise an optical element 10b. The optical element 10b may comprise one or more lenses (Fig. 3A), one or more mirrors (Fig. 3B), one or more diffractive optical elements (DOEs) (Fig. 3C), or a combination of these. For example, one or more lenses and one or more mirrors may be combined to utilize total internal reflection (Fig. 3D). Other combinations are also possible, including combinations of the same type of deflection element (e.g. two or more lenses, two or more mirrors, two or more DOEs). The radiation pattern may be adjusted by moving and/or rotating a part of the optical element, e.g. moving one lens/mirror/DOE while maintaining the position of another lens/mirror/DOE.

In this way, the propagation of the radiation beam 11 may be adjusted in various way. For example, the radiation control unit 30 may be adapted to adjust the direction (along the centerline 'c') and/or spread angle of the radiation beam and/or the focus of the beam, in reaction to an input from the detection unit.

Alternatively or additionally, the radiation pattern may be adjusted by altering the radiation emitted from the radiation source 10a, as shown in Figs. 4A and 4B, which may be implemented in the embodiments discussed above.

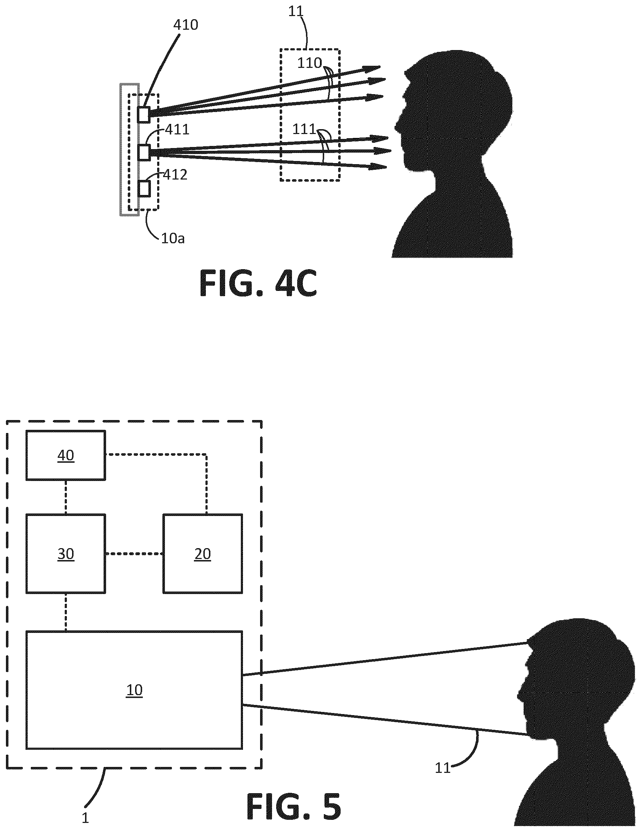

As shown in Fig. 4A, a multiple-element emitter may be used. In the embodiment shown, the radiation source 10a of the radiation unit 10 comprises a plurality of radiation elements 410, 411, 412, each driven by a driving current. Although the figure shows only three radiation elements, any other number for the radiation elements may be implemented. In this embodiment, the radiation control unit 30 (not shown in Fig. 4) is adapted to adjust the driving current for individual radiation elements in reaction to an input from the detection unit, so as to adjust the radiation pattern of the radiation beam 11 (which in this embodiment is the collection of radiations 110, 111, 112 from the individual radiation elements 410, 411, 412). Each radiation element in the multiple-element emitter may be arranged to map to an effective illuminated region. For example, when the predetermined area is within the effective region, that radiation element is switched on. When the predetermined area is not within the effective region, that radiation element is not switched on (e.g. switched off by default). This enables the electro-optical device to project the radiation beam 11 to the desired location without requiring any moving parts.

In an embodiment, the radiation unit comprises a plurality of LEDs, and the radiation control unit is adapted to switch on or switch off a subset of the plurality of LEDs in reaction to an input from the detection unit, so as to adjust the radiation pattern of the radiation beam. This enables switching on and switching off subset of the plurality of LEDs based on the detection of the predetermined area. Each subset of LEDs may comprise one or more LEDs, and different subsets may comprise different numbers of LEDs. Different subsets of LEDs may be used for projecting the radiation beam towards different users.

VCSEL arrays (vertical-cavity surface emitting laser arrays) is another suitable choice. Compared to LEDs, VCSEL arrays have an advantage of the relatively narrow radiation beam they provide. For example, a commercially available VCSEL array product, APS6401010002 from II-VI, Inc., can produce a beam with 25 degrees.

A VCSEL array typically contains a plurality of individual emitters. Each individual emitter may be provided with an integrated deflection member such as mirror or lens, to set a beam direction. Typically, each deflection member is fixed in position but configured differently for each individual emitter, so each emitter irradiates in a predetermined direction. The individual emitter could be switched on or off so as to only irradiate an object of interest (within the broad far field pattern). This makes it possible to switch on and off these individual emitters based on the input from the detection unit 20.

Fig. 4B shows an embodiment of the radiation unit 10 using a VCSEL array comprising a plurality of emitters. In the embodiment shown, the VCSEL array has a plurality of active regions 410a, 411a, 412a (typically in the form of quantum wells in a semiconductor laser diode) for generating radiations, which may function as the radiation source 10a, and a substrate 420 forming a plurality of microlenses 410b, 411b, 412b, which may function as (part or the entirety of) the optical element 10b. The VCSEL array may further comprise a submount for accommodating the plurality of emitters. The microlenses 410b, 411b, 412b are shaped and/or positioned to direct the laser radiations in various different directions. In the embodiment shown, the microlens 411b is co-centered with the corresponding active region 411a (so the laser radiation 111 generated by the active region 411a is not deflected), while the microlenses 410b and 412b are de-centered with respective to the corresponding active regions 410a and 412b in different directions (so the laser radiations 110 and 112 generated by the active regions 410a and 412a are deflected into the designed directions). By switching on and off the individual emitters (active regions), the radiation pattern can be controlled. This way, no moving part is required. Alternatively, a more flexible beam control may be achieved by also moving and/or rotating the microlenses 410b, 411b, 412b in the VCSEL array relatively to the active regions 410a, 411a, 412a. A simple adjustment may be achieved by moving the array of microlenses 410b, 411b, 412b (as shown in arrows b in Fig. 4B), by moving the array of active regions 410a, 411a, 412a (as shown in arrows a in Fig 4B), or both. It is also possible to manufacture the microlenses 410b, 411b, 412b as separate parts, and control these elements individually, e.g. using so-called Microelectromechanical systems (MEMS).

For example, as shown in Fig. 4C, for each radiation element 410, 411, 412, the radiation control unit 30 (not shown in Fig. 4C) may be adapted to switch on the radiation element when the predetermined area is within the effective region, and does not switch on the radiation element when the predetermined area is not within the effective region. In the embodiment shown, the predetermined area (the user's face) falls outside the illumination region of the radiation element 412, so this element is turned off. The predetermined area falls within the illumination regions of the radiation elements 410, 411, so these elements are turned on. Note that this schematic figure only shows three radiation elements for simplicity. In practice a LED or VCSEL array contains many more radiation elements.

### Multi-user handling and personal dose control

Fig. 5 shows an embodiment in which the electro-optical device 1 further comprises a timer 40. This embodiment may comprise one or more measures described above in the context of Figs. 1-4.

Research demonstrates that the benefit of PBM effect varies in dependence on the accumulated dose per day. When the accumulated dose is too small, the benefit is insignificant. When the accumulated dose is too much, the beneficial PBM effect also deteriorates and may even cause damages. It is therefore preferable to control the accumulated dose per day within a certain amount (e.g. 0.01-50 J/cm², preferably in the range 0.1-20 J/cm², more preferably in the range 1-10 J/cm², more preferably in the range 4-7 J/cm²).

In line with this, the timer 40 may start in reaction to an input from the detection unit 20. For example, when the detection unit 20 detects or recognizes a face, it may instruct the timer 40, or to instruct the radiation control unit 30 to start the timer. Once the timer expires, indicating that a sufficient amount of dose level has been accumulated for a day (e.g. 0.01-50 J/cm²), the radiation control unit 30 then stops the irradiation of the radiation beam 11 from the radiation unit 10. The timer 40 may be set in accordance with a predetermined expiration time (e.g. two hours), or such that the timer expires after a predetermined dose level (e.g. 0.01-50 J/cm²) in the predetermined area is exceeded.

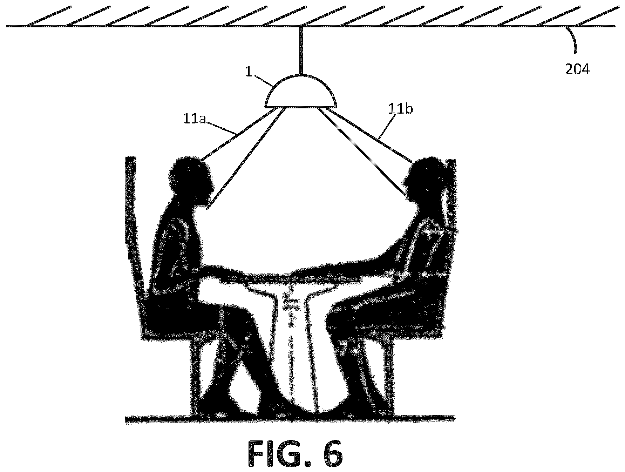

Fig. 6 shows an embodiment of the electro-optical device 1, in which the dose control described in Fig. 5 can be achieved on a user-by-user basis.

In this embodiment, the detection unit is adapted to recognize and distinguish between individual users (e.g. using facial recognition instead of facial detection). The radiation pattern is such that the radiation beam 11 is directed to all the users individually. In the embodiment shown, the radiation beam 11 comprises a plurality of beamlets 11a and 11b, each directed to a predetermined area (e.g. face) of a recognized user. The same applies when there are more than two users, in which case there are more than two beamlets. Alternatively or additionally, the radiation beam 11 may rotate between the users under the control of the radiation control unit 30 (not shown in Fig. 6). In this way, for every user a peak irradiation intensity above 0.1 mW/cm², preferably above 1 mW/cm², in the predetermined area can be achieved at the same time or at different times. By taking face detection a step further towards face recognition in this embodiment, the electro-optical device can easily keep track of each user even if the users happen to move or swap positions.

For each individual user, the electro-optical device 1 may start a timer upon recognizing the user by the detection unit 20, and the radiation control unit 30 may adjust the radiation pattern upon expiration of each individual timer, so that the radiation beam 11 is no longer directed to a user whose accumulated dose has exceeded a certain threshold. For example, in the embodiment shown in Fig. 6, when the user on the left has obtained an accumulated dose exceeding the threshold, the radiation control unit 30 could switch off the beamlet 11a (e.g. by switching off corresponding LED or VCSEL emitters, etc).

The radiation control unit may further pause the corresponding timer when the user is no longer recognized (e.g. when the user leaves the room, is temporarily blocked, or moves too far away), and resume the timer when the user is recognized again. As the electro-optical device 1 recognizes each specific user (using racial recognition in this embodiment), this enables the electro-optical device to maintain an accurate dose control, without being interrupted by some users happening to disappear for some time during operation.

In the embodiment shown in Fig. 6, the electro-optical device 1 is incorporated in a general lighting device, but this embodiment is equally applicable to any embodiments of the electro-optical device 1 described above, such as a desktop monitor or a television described above.

Certain embodiments described above involve logic operations or calculations. These may be implemented using software (e.g., code embodied on a machine-readable medium or in a transmission signal), hardware, or a combination of these. In software implementation, one or more processors may be used. A hardware implementation may involve the use of dedicated circuitry or logic that is configured to perform certain operations. For example, a hardware module may be a programmable logic device such as a field programmable gate array (FPGA) or an ASIC. It will be appreciated that the decision to implement using software, hardware, or combination of these, may be driven by cost and time considerations.

The descriptions above are intended to be illustrative, not limiting. It will be apparent to the person skilled in the art that alternative and equivalent embodiments of the invention can be conceived and reduced to practice, without departing from the scope of the claims set out below.

## Claims

1. An electro-optical device (1), comprising:
a radiation unit (10) adapted to emit a radiation beam (11) having a peak emission wavelength between 610-1400nm;
a detection unit (20) adapted to detect a predetermined area of the body of a user; and
a radiation control unit (30) for receiving an input from the detection unit,
wherein the electro-optical device (1) is adapted to direct the radiation beam (11) towards the predetermined area using the radiation control unit (30), such that the radiation beam has a peak irradiation intensity above 0.1 mW/cm² at a surface of the body of the user in the predetermined area.

2. The electro-optical device (1) according to claim 1, wherein the radiation control unit (30) is adapted to control the radiation unit to adaptively adjust a radiation pattern of the radiation beam (11) in dependence on an input from the detection unit.

3. The electro-optical device (1) according to claim 1 or 2, wherein the predetermined area covers at least a portion of the face of the user.

4. The electro-optical device (1) according to any of the preceding claims, wherein the radiation unit (10) is adapted to project the radiation beam (11) in a first direction, and the radiation control unit is adapted to adjust the first direction and/or spread angle of the radiation beam in reaction to an input from the detection unit, so as to adjust a radiation pattern of the radiation beam (11).

5. The electro-optical device (1) according to any of the preceding claims, wherein the radiation unit (10) comprises an optical element (10b) comprising one or more lenses, and/or one or more mirrors, and/or one or more diffraction optical elements, and wherein the radiation control unit (30) is adapted to move and/or rotate a part of the optical element to adjust the first direction of the radiation beam.

6. The electro-optical device (1) according to any of the preceding claims, wherein the radiation unit (10) comprises a plurality of radiation elements (410, 411, 412), each driven by a driving current, and wherein the radiation control unit is adapted to adjust the driving current for individual radiation elements in reaction to an input from the detection unit, so as to adjust a radiation pattern of the radiation beam (11).

7. The electro-optical device (1) according to any of the preceding claims, wherein the radiation unit comprises a plurality of LEDs, and the radiation control unit is adapted to switch on or switch off a subset of the plurality of LEDs in reaction to an input from the detection unit to adjust a radiation pattern of the radiation beam (11).

8. The electro-optical device (1) according to any of the preceding claims, wherein the radiation unit comprises a vertical cavity surface emitting laser (VCSEL) array having one or more radiation elements, each radiation element having an effective illumination region, wherein, for each radiation element, the radiation control unit (30) is adapted to switch on the radiation element when the predetermined area is within the effective illumination region, and does not switch on the radiation element when the predetermined area is not within the effective illumination region.

9. The electro-optical device (1) according to any of the preceding claims, wherein the detection unit is adapted to determine a distance between the electro-optical device (1) and the predetermined area generate an output in dependence on said distance, and the radiation control unit is adapted to react to the detection unit in dependence on the output of the detection unit (20).

10. The electro-optical device (1) according to any of the preceding claims, wherein the electro-optical device is adapted to be placed on a desk or a table.

11. The electro-optical device (1) according any of the preceding claims, wherein the electro-optical device (1) is mounted on a ceiling or a wall, and/or wherein the electro-optical device is incorporated in a portable user equipment and/or a display apparatus.

12. The electro-optical device (1) according to any of the preceding claims, wherein the electro-optical device is incorporated in a general lighting apparatus, wherein the radiation unit is adapted to project the radiation beam in a spread angle within 2×30°, preferably within 2×25°, more preferably within 2×20°, more preferably within 2×15°, more preferably within 2×10°.

13. The electro-optical device (1) according to any of the preceding claims, wherein the radiation unit is adapted to project the radiation beam in a spread angle within +/- 30°, preferably within +/- 25°, more preferably within +/- 20°, more preferably within +/- 15°, more preferably within +/- 10° about a center beam line.

14. The electro-optical device (1) according to any of the preceding claims, wherein the detection unit is adapted to recognize and distinguish between individual users, and for each recognized user, the radiation control unit is adapted to direct the radiation beam towards a predetermined area in the body of said user by adjusting the radiation pattern, such that in each predetermined area for each user, the radiation beam has a peak irradiation intensity above 0.1 mW/cm².

15. The electro-optical device (1) according to claim 14, wherein, for each recognized user, the electro-optical device is adapted to start a timer upon recognizing the user by the detection unit, and the radiation control unit is adapted to adjust the radiation pattern upon expiration of each individual timer on a user-by-user basis.
